# EUROPÄISCHE PATENTSCHRIFT

(11) **EP 3 738 629 B1**
(45) Veröffentlichungstag und Bekanntmachung des Hinweises auf die Patenterteilung: **08.03.2023**
(21) Anmeldenummer: 19174929.0
(22) Anmeldetag: 16.05.2019
(51) Int. Cl.: A61M 5/32, A61M 5/315

(54) **MEDIZINISCHE SPRITZE MIT NADELSCHUTZ**
MEDICAL SYRINGE WITH NEEDLE SHIELD
SERINGUE MÉDICALE À PROTECTION D'AIGUILLE

(43) Veröffentlichungstag der Anmeldung: 18.11.2020
(73) Patentinhaber: Trenta2 S.r.l., 39100 Bozen (IT)
(72) Erfinder: GALLMETZER, Dietrich, 39018 Terlan (IT)
(74) Vertreter: Tergau & Walkenhorst Patentanwälte PartGmbB

(56) Entgegenhaltungen:
- EP-A2- 0 340 899
- WO-A1-2011/075760
- IT-A1- UB20 160 423
- US-A- 4 804 370
- US-A1- 2001 021 821
- US-A1- 2002 161 340

## Beschreibung

Die Erfindung betrifft eine medizinische Spritze mit Nadelschutz mit einem über einen Betätigungsstößel im Innenraum eines Spritzenkörpers verschiebbaren Kolben.

Zur Vermeidung oder Verringerung von Kontaminations- oder Verletzungsrisiken nach dem Gebrauch von Medikamentenspritzen und insbesondere zur Vermeidung des mehrfachen Gebrauchs von Spritzennadeln durch verschiedene Nutzer finden Spritzen mit so genannten Einzieh- oder Retraktionssystemen für die Spritzennadel zunehmend Verwendung. Bei solchen insbesondere medizinischen Spritzen, auch als "Spritze mit (passivem) Nadelschutz" bezeichnet, wird die Spritzennadel nach Abgabe des in der Spritze vorgehaltenen Wirkstoffs in den Spritzenkörper eingezogen und von diesem vollständig umschlossen. Ein Zugang zur Spritze und damit ein Verletzungsrisiko, oder auch das Risiko mehrfachen Gebrauchs derselben Nadel, kann damit weitgehend ausgeschlossen werden. Derartige Spritzen mit passivem Nadelschutz sind beispielsweise aus der EP 1 284 769 B1, der EP 0 720 491 B1, der EP 0 680 347 B1, der EP 0 340 899 A2, der US 4 804 370, der IT UB20 160 423 A1 oder der EP 1 764 127 B1 bekannt.

Ganz allgemein bei medizinischen Spritzen, und somit auch bei Spritzen mit Nadelschutz der genannten Art, besteht das Bedürfnis, beim Gebrauch der Spritze den darin enthaltenen Wirkstoff möglichst vollständig abzugeben und den Verbleib nicht genutzter Restmengen des Wirkstoffs innerhalb der Spritze möglichst weitgehend zu minimieren. Der Erfindung liegt nunmehr die Aufgabe zugrunde, eine Spritze mit Nadelschutz der oben genannten Art anzugeben, mit der diesem Bedürfnis Rechnung getragen ist.

Diese Aufgabe wird erfindungsgemäß gelöst, durch eine Spritze mit Nadelschutz gemäß Anspruch 1.

Vorteilhafte Ausgestaltungen der Erfindung sind Gegenstand der Unteransprüche und/oder der nachfolgenden Figurenbeschreibung entnehmbar.

Die Erfindung geht von der Überlegung aus, dass die vollständige Ausbringung des Wirkstoffs infolge der Verschiebung des Kolbens innerhalb des Sprizengehäuses zu dessen distalem Ende hin im Wesentlichen durch das so genannte Totvolumen begrenzt wird, also geometriebedingt vorliegende Taschen oder Toträume innerhalb des Gehäuses, von denen aus der Wirkstoff bei vollständig verschobenem Kolben nicht mehr über die Nadel ausströmen kann. Für die angestrebte Minimierung der nach der Nutzung der Spritze in deren Gehäuse verbleibenden Wirkstoffreste sollten diese Toträume somit besonders klein gehalten werden, und es sollten gezielt Abströmkanäle bereitgestellt werden, über die der Wirkstoff auch bei weit vorgeschobenem Kolben noch dem Eintrittsende der Nadel zuströmen kann. Dies kann gerade bei Spritzen mit Nadelschutz problematisch sein, da bei derartigen Systemen die Nadel bei weit vorgeschobenem Kolben erfasst und anschließend mit dem Kolben in den Innenraum des Spritzengehäuses zurückgezogen werden muss. Das System zur Erfassung und Mitnahme der Nadel sollte daher auch bei weit vorgeschobenem Kolben eine möglichst weitgehende Zuströmung des Wirkstoffs zum inneren Nadelende ermöglichen. Dies wird erreicht, indem die Nadel über einen Bügel erfasst wird, der in seinen seitlichen Bereichen Zuströmflächen für den Wirkstoff freilässt, über den dieser der Nadel auch dann noch zuströmen kann.

Vorteilhafterweise ist der Nadelhalter dabei als Kunststoffteil, vorzugsweise aus Polypropylen, gefertigt. Ganz besonders bevorzugt ist dabei als Basismaterial im Hinblick auf die erwarteten erforderlichen Haltekräfte und die bei bestimmungsgemäßem Gebrauch erwarteten mechanischen Belastungen, aber auch im Hinblick auf zulassungsbedingte Erfordernisse das unter der Bezeichnung "Bormed^{™}" (HD810MO, ISO 10993 Information (Biocompatibility)) erhältliche Polypropylen vorgesehen.

Vorteilhafterweise ist der Nadelhalter von einem Kolbenmantel umgeben, der derart geformt ist, dass er bei eingebrachtem Nadelhalter seitlich des Haltebügels eine Anzahl von Zuströmkanälen für den Wirkstoff freilässt. Der Kolbenmantel besteht dabei bevorzugt aus Gummi.

Ein Ausführungsbeispiel der Erfindung wird anhand einer Zeichnung näher erläutert. Darin zeigen:
- Fig. 1: eine medizinische Spritze mit Nadelschutz,
- Fig. 2: eine Betätigungseinheit der Spritze nach Fig. 1 in seitlicher Ansicht,
- Fig. 3: eine Deckelplatte der Spritze nach Fig. 1,
- Fig. 4: die Deckelplatte nach Fig. 3 mit angesetztem Betätigungsstößel,
- Fig. 5: eine Sequenz von Teilschnitten der Spritze gemäß Fig. 1,
- Fig. 6: eine zur Verwendung in der Spritze gem. Fig. 1 vorgesehene, in einen Nadelhalter eingesteckte Nadel in seitlicher Ansicht,
- Fig. 7: die Nadel gem. Fig. 6 in perspektivischer Ansicht,
- Fig. 8: einen Kolben der Spritze gem. Fig. 1,
- Fig. 9: einen Betätigungsstößel der Spritze gem. Fig. 1,
- Fig. 10: eine alternative Ausführungsform einer medizinischen Spritze mit Nadelschutz,
- Fig. 11: eine Kartuschen- oder Patroneneinheit der Spritze gem. Fig. 10,
- Fig. 12: eine Verschlusskappe der Kartuscheneinheit gem. Fig. 11,
- Fig. 13: den Nadelkopf der Spritze gem. Fig. 10 in Explosionsdarstellung,
- Fig. 14: einen Spritzenrahmen der Spritze gem. Fig. 10,
- Fig. 15: die Kartuscheneinheit gem. Fig. 11 unmittelbar vor (Fig. 15a) und nach (Fig. 15b) der Einbringung in den Spritzenrahmen gem. Fig. 14,
- Fig. 16: den Verbindungsbereich zwischen Kolben und Betätigungsstößel der Spritze gem. fig. 10 im Längsschnitt,
- Fig. 17: die Spritze gem. Fig. 10 bei Anbringung und Freilegen der Nadel,
- Fig. 18: die Spritze gem. Fig. 10 nach Ausbringung des Wirkstoffs und Retraktion der Nadel,
- Fig. 19: den Kartuschenkörper der Spritze gem. Fig. 10 mit darin befindlicher eingezogener Nadel, und
- Fig. 20: eine weitere alternative Ausführungsform einer medizinischen Spritze mit Nadelschutz und Doppelkammersystem im Längsschnitt.

Gleiche Teile sind in allen Figuren mit denselben Bezugszeichen versehen.

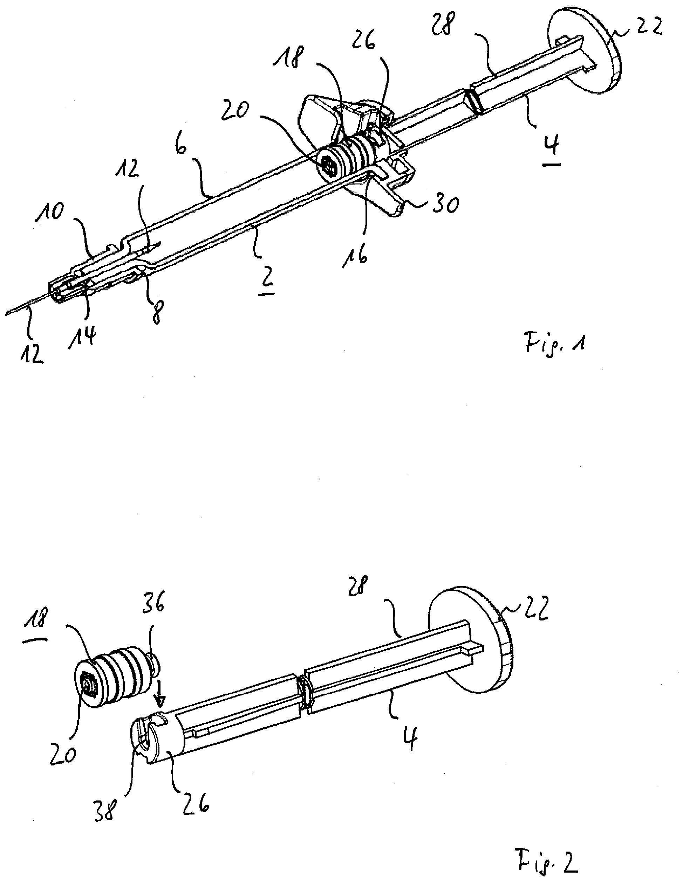

Die medizinische Spritze 1 mit Nadelretraktion gemäß Fig. 1 umfasst im Wesentlichen zwei Baugruppen, nämlich einerseits eine Kartuschen- oder Patroneneinheit 2 und andererseits einen Betätigungsstößel 4. Im Ausführungsbeispiel ist die medizinische Spritze 1 dabei zweikomponentig aufgebaut, wobei die beiden genannten Baugruppen separate, auf die nachstehend beschriebene Weise miteinander verbindbare Komponenten bilden. Alternativ könnte die Spritze aber auch einkomponentig ausgeführt sein, wobei die beiden genannten Baugruppen von vornherein miteinander verbunden sind und die Unterscheidung in die beiden Baugruppen lediglich funktional erfolgt.

Die Kartuschen- oder Patroneneinheit 2 bildet die eigentliche Spritze und umfasst einen entsprechend einer herkömmlichen Bauweise zylindrisch oder rohrförmig ausgeführten, ein Spritzengehäuse bildenden Hohlkörper 6, der zur Aufnahme des medizinischen Wirkstoffs vorgesehen ist. Am vorderen oder distalen Ende 8 des Hohlkörpers 6 ist ein Nadelhalter 10 befestigt, in dem die zur Injektion des Wirkstoffs vorgesehene Hohlnadel 12 in einer Lagermuffe 14 gelagert ist. Der Nadelhalter 10 könnte dabei einstückig mit dem das Spritzengehäuse bildenden Hohlkörper 6 ausgeführt sein. Im Ausführungsbeispiel ist der Nadelhalter 10 aber in als eigenständig erfinderisch angesehener Ausführung als separates Bauteil ausgestaltet. Der Nadelhalter 10 ist dabei vorliegend auf den das Spritzengehäuse bildenden Hohlkörper 6 bzw. den Spritzenkonus aufgesteckt oder aufsteckbar, könnte aber auch mittels eines Gweindes, beispielsweise eines Luer-Gewindes, anschraubbar ausgeführt sein.

Die Nadel 12 ist zur Vermeidung von Verletzungen oder dergleichen von einer nicht näher dargestellten abnehmbaren Schutzkappe umgeben, die vor dem Einsatz der Spritze 1 entfernt wird. Das hintere oder proximale Ende 16 des das Spritzengehäuse bildenden Hohlkörpers 6 ist hingegen von einem in seinen Außenabmessungen passgenau an die Innenkontur des Hohlkörpers 6 angepassten, innerhalb des Hohlkörpers 6 verschiebbaren Kolben 18 verschließbar. Im in Fig. 1 gezeigten Zustand, also bei mit der Kartuschen- oder Patroneneinheit 2 verbundenem Betätigungsstößel 4 und vor der Aufnahme oder Ausbringung des medizinischen Wirkstoffs, ist dieser somit im Inneren des endseitig durch den Kolben 18 verschlossenen Hohlkörpers 6 eingeschlossen. An seiner dem Innenraum des Hohlkörpers 6 zugewandeten Endfläche weist der Kolben 18 mittig ein Aufnahmeloch 20 für die Nadel 12 auf.

Der Antriebs- oder Betätigungsstößel 4 umfasst einen einerseits endseitig mit einer Drückerplatte 22 versehenen und andererseits an seinem der Drückerplatte 22 gegenüberliegenden Ende 24 mit einem zur Verbindung mit dem Kolben 18 vorgesehenen Kuppelelement 26 versehenen Schaft 28. Mit seinem sich zwischen Drückerplatte 20 und Kuppelelement 26 erstreckenden Schaft 28 ist der Betätigungsstößel 4 im montierten Zustand der Komponenten durch eine Deckelplatte 30 hindurch geführt und in dieser in seiner Längsrichtung verschiebbar gelagert.

Der Kolben 18 könnte einstückig mit dem Betätigungsstößel 4 ausgeführt sein. Im Ausführungsbeispiel sind diese Bauteile jedoch separat ausgeführt. Wie der Darstellung in Fig. 2 entnehmbar ist, weist der Kolben 18 zur Verbindung mit dem Schaft 28 des Betätigungsstößels 4 einen endseitig angeformten, mit einer umlaufenden Rille versehenen Befestigungsstift 36 auf. Korrespondierend zu diesem und in der Dimensionierung an diesen angepasst ist das an den Schaft 28 endseitig angeformte Kuppelelement 26 mit einem einseitig offenen Langloch 38 versehen. In dieses ist der Befestigungsstift 36 seitlich einschiebbar, so dass die Seitenkante des Langlochs 38 in die umlaufende Rille des Befestigungsstifts 36 eingreift und damit in Längsrichtung gesehen ein Formschluss zwischen diesen Komponenten entsteht.

Die medizinische Spritze 1 ist durch die genannten Komponenten und Bauteile mit einem in der Art eines Retraktionssystems ausgeführten Nadelschutz versehen. Damit wird bezweckt, dass nach der Benutzung der Spritze 1, also nach der Ausgabe des im das Spritzengehäuse bildenden Hohlkörper 6 vorgehaltenen Wirkstoffs über die Nadel 12, diese in das Spritzengehäuse derart hineingezogen wird, dass sie vom Spritzengehäuse vollständig umschlossen wird. Damit soll eine unbeabsichtigte Berührung der benutzten Nadel 12, beispielsweise durch Hilfs- oder Pflegepersonal, und somit das Verletzungs- und Kontaminationsrisiko besonders gering gehalten oder möglichst ganz ausgeschlossen werden.

Dafür ist grundsätzlich der folgende Ablauf beim Einsatz und der Handhabung der genannten Komponenten vorgesehen:
Die Spritze 1 könnte grundsätzlich mit vorbefüllter Kartuschen- oder Patroneneinheit 2 eingesetzt werden. In diesem Fall würde die mit dem Wirkstoff befüllte Kartuschen- oder Patroneneinheit 2 mit bereits in den Hohlkröper 6 eingebrachtem und dessen Innenraum verschließendem Kolben 18 bereitgestellt. Als ersten Schritt bei der Benutzung wird dann gemäß der in Fig. 2 dargestellten Weise der Betätigungsstößel 4 endseitig mit dem Kolben 18 verbunden, und das System ist zur Ausgabe des Wirkstoffs bereit.

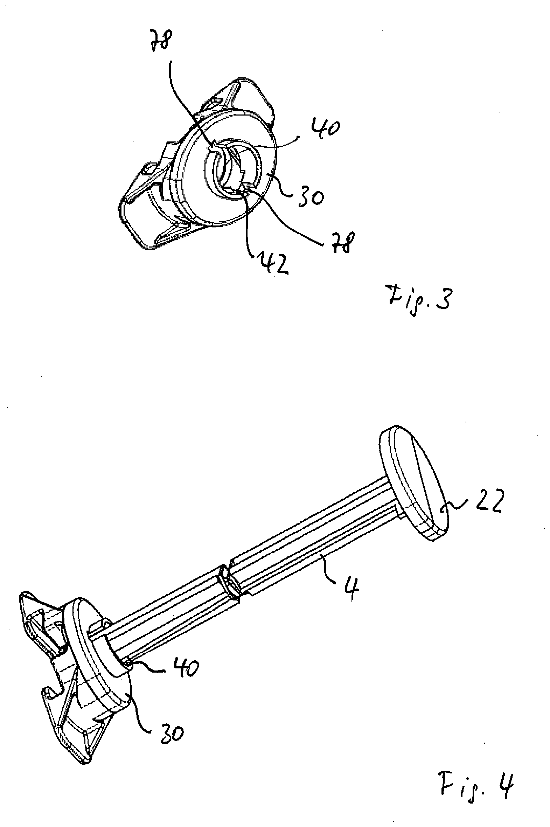

Im Ausführungsbeispiel ist jedoch vorgesehen, dass die Spritze 1 leer einsatzfähig gemacht und durch Aufziehen mit dem Wirkstoff befüllt wird. Dazu wird zunächst, wie dies der vergrößerten Darstellung der Deckelplatte 30 von schräg oben gem. Fig. 3 und der Deckelplatte 30 mit angesetztem Betätigungsstößel 4 von schräg oben gem. Fig. 4 entnehmbar ist, der den Kolben 18 tragende Betätigungsstößel 4 in das hierfür vorgesehene Aufnahmeloch 40 in der Deckelplatte 30 eingeschoben, so dass der Kolben 18 in den Innenraum des Hohlkörpers 6 eingebracht wird. Das Aufnahmeloch 40 mit seinem in der Art einer Führungskulisse ausgestalteten Außenrand 42 und korrespondierend dazu der Querschnitt des Schafts 28 des Betätigungsstößels 4 sind dabei derart ausgestaltet, dass die Einbringung in einer vorgegebenen rotatorischen Ausrichtung des Betätigungsstößels 4 relativ zum Hohlkörper 6 bzw. dessen Deckelplatte 30 erfolgt.

Anschließend wird der Betätigungsstößel 4 unter Beibehaltung dieser rotatorischen Ausrichtung - dies wird durch eine in Längsrichtung gesehen gleichbleibende Querschnittsgeometrie des Schafts 28 erreicht - vom Bediener gedrückt, so dass sich der Kolben 18 innerhalb des Hohlkörpers 6 auf dessen distales Ende 8 hinzu bewegt. Sobald dabei eine vorgegebene Endposition erreicht wird, bei der der Kolben 18 im Sinne des unten beschriebenen Mechanismus noch ausreichend weit von der Nadel 12 entfernt ist, wird diese Bewegung durch einen durch den Schaft 28 gebildeten Anschlag gestoppt. Der Anschlag wird dabei durch eine in der Art einer Stufe oder Kante ausgeführte, in Längsrichtung gesehen sprunghafte Querschnittsveränderung oder - aufweitung des Schafts 28 gebildet, so dass der Schaft 28 in Längsrichtung nicht weiter durch den Außenrand 42 des Aufnahmelochs 40 hindurch geführt werden kann.

Der Querschnitt des Schafts 28 und korrespondierend dazu die die Führungskulisse bildende Kontur des Außenrands 42 des Aufnahmelochs 40 sind dabei derart ausgeführt, dass bei Erreichen der genannten Endposition des Kolbens 18 innerhalb des Hohlkörpers 6 das rotatorische Gesperre zwischen Betätigungsstößel 4 und Deckelplatte 30 zumindest in gewissem Umfang aufgehoben wird und der Stößel 4 in der Deckelplatte 30 um seine Längsachse um einen vorgegebenen Verdrehwinkel, vorzugsweise etwa 90°, verdrehbar ist. Durch diese Verdrehung wird erreicht, dass einerseits der bisher vorliegende, ein weiteres Verschieben des Kolbens 18 in Richtung zum distalen Ende 8 hin behindernde Anschlag entriegelt wird, so dass der Kolben 18 nunmehr vollständig bis in die finale Position innerhalb des Hohlkörpers 6 geschoben werden könnte. Andererseits wird durch diese Rotation aber auch - wiederum erreicht durch eine geeignete Gestaltung des Querschnitts des Schafts 28 und angepasst daran der Kontur des Außenrands 42 - ein neuer Anschlag "nach oben hin" oder zum proximalen Ende 16 des Hohlkörpers 6 hin gebildet, so dass über einen hierdurch vorgegebenen Endpunkt hinaus der Betätigungsstößel 4 und insbesondere der an diesem endseitig befestigte Kolben 18 nicht aus dem Hohlkörper 6 herausgezogen werden kann.

In dieser Position wird nunmehr das freie Ende der Nadel 12 in ein externes Reservoir des Wirkstoffs eingebracht, und anschließend wird der Kolben 18 mittels des Betätigungsstößels 4 im Hohlkörper 6 zurückgezogen. Dabei wird der Wirkstoff über die Nadel 12 angesaugt und der Innenraum des Hohlkörpers 6 somit befüllt.

Nachdem die Spritze 1 auf eine der genannten Weisen einsatzfertig gemacht wurde, wird die Nadel 12 zur Verabreichung des medizinischen Wirkstoffs geeignet am Patienten positioniert, so dass sie an geeigneter Stelle die Haut des Patienten durchstößt. Die Haltekreft der Nadel 12 in der Lagermuffe 14 ist dabei, insbesondere durch geeignete Dimensionierung der Komponenten und/oder die Wahl der Materialpaarung, derart vorgegeben, dass die Nadel 12 sicher in ihrer Position in der Lagermuffe 14 verbleibt, wenn der Bediener durch Handhabung am Hohlkörper 6 die Nadel 12 durch die Haut des Patienten hindurchsticht.

Anschließend wird der Betätigungsstößel 4 vom Bediener gedrückt, so dass sich der Kolben 18 innerhalb des Hohlkörpers 6 auf dessen distales Ende 8 hin bewegt und dabei den medizinischen Wirkstoff der Nadel 12 zuführt und über diese ausbringt. Kurz vor vollständiger Ausbringung des Wirkstoffs, also kurz vor vollständiger Entleerung des Innenraums des Hohlkörpers 6, erreicht der Kolben 18 in der Nähe des distalen Endes 8 des Hohlkörpers 6 das in den Innenraum ragende Ende der Nadel 12, so dass diese bei weiterer Bewegung in das dafür vorgesehene Aufnahmeloch 20 eindringt. Nach vollständiger Ausbringung des Wirkstoffs erreicht der Kolben 18 dann seine Endposition unmittelbar am distalen Ende 8 des Hohlkörpers 6 und umschließt dabei den in das Aufnahmeloch 20 hineinragenden Teil der Hohlnadel 12. Diese Einbringung des entsprechenden Teilbereichs der Hohlnadel 12 in das Aufnahmeloch 20 und die dadurch erreichte Verbindung der Nadel 12 mit dem Kolben 18 wird vorliegend auch als "connecting" bezeichnet.

Nach der Verabreichung des Wirkstoffs und insbesondere nach vollständiger Entleerung des Hohlkörpers 6 wird vom Bedierer die Drückerplatte 22 und mit dieser auch der Betätigungsstößel 4 insgesamt wieder zurückgezogen. Damit wird auch der über das Kuppelelement 26 mit dem Schaft 28 des Betätigunsstößels 4 verbundene Kolben 18 mitgenommen und innerhalb des Hohlkörpers 6 vom distalen Ende 8 weg zum proximalen Ende 16 hin gezogen. Dabei nimmt er seinerseits die eingefasste Nadel 12 mit und zieht diese in den Hohlkörper 6 hinein, so dass diese im Endzustand vollständig innerhalb des Hohlkörpers 6 positioniert ist.

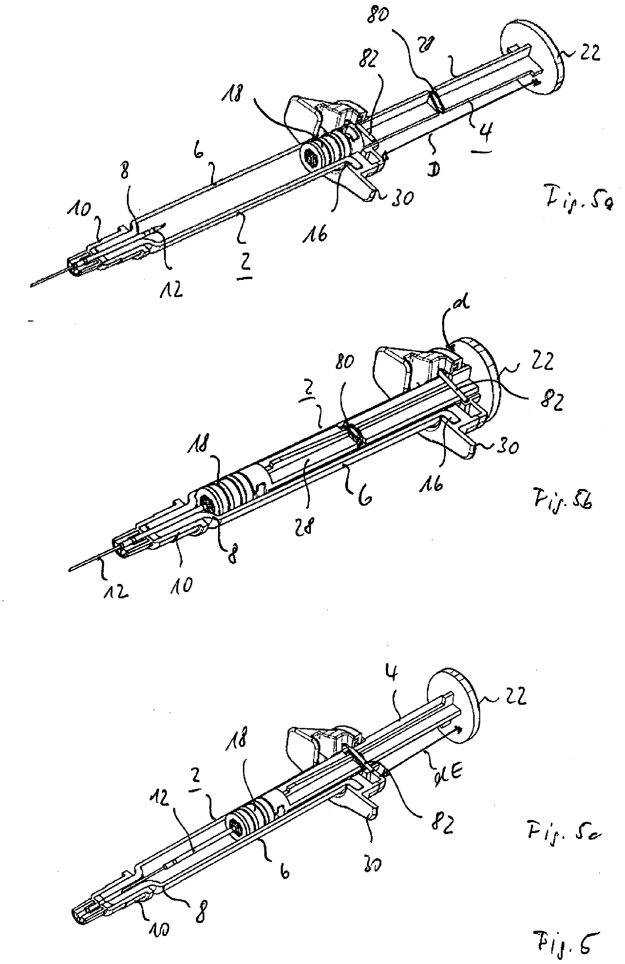

Zur besseren Verdeutlichung ist diese Abfolge in der Sequenz von Teilschnitten in Fig. 5 dargestellt. Darin zeigt Fig. 5a die Spritze 1 vor Verabreichung des im Innenraum des Hohlkörpers 6 vorgehaltenen Wirkstoffs. Der im Innenraum des Hohlkörpers 6 geführte Kolben 18 befindet sich dabei an seinem Anschlag an der am proximalen Ende 16 des Hohlkörpers 6 angeordneten Deckelplatte 30. Der Betätigungsstößel 4 ist dementsprechend voll ausgefahren, und die Drückerplatte 22 befindet sich im maximalen Abstand D von der Deckelplatte 30. Die im Nadelhalter 10 gehaltene Hohlnadel 12 ist in diesem Zustand einsatzbereit ausgefahren.

Ausgehend von diesem Zustand wird - insbesondere bei in die Haut des Patienten eingestochener Nadel 12 - der Wirkstoff ausgebracht, indem der Kolben 18 von seiner in Fig. 5a gezeigten Ausgangsposition bis zu der in Fig. 5b gezeigten Endposition unmittelbar benachbart zum distalen Ende 8 des Hohlkörpers 6 hin verschoben wird. Zu diesem Zweck wird der Betätigungsstößel 4 in den Hohlkörper 6 hineingeschoben bis hin zur Endposition, in der die Drückerplatte 22 den minimalen Abstand d von der Kupplungsplatte 30 einimmt. Die im Nadelhalter 10 gehaltene Hohlnadel 12 ist in diesem Zustand immer noch ausgefahren, ist aber bereits in die Aufnahmeöffnung 20 des Kolbens 18 eingedrungen und wird mit ihrem in den Innenraum des Hohlkörpers 6 ragenden Ende vom Kolben 18 umfasst und mit diesem mechanisch verbunden.

Nach Erreichen der in Fig. 5b gezeigten Endposition des Kolbens 18 bzw. des Betätigungsstößels 4 und der damit erreichten vollständigen Ausgabe des Wirkstoffs wird der Betätigungsstößel 4 vom Bediener wieder zurückgezogen bis zur Endposition, die in Fig. 5c gezeigt ist. In dieser Endposition befindet sich die Drückerplatte 22 in einer Position mit dem Endabstand dE von der Deckelplatte 30. Entsprechend hat sich der Kolben 18 dabei mitbewegt, so dass er sich in dieser Position in einer mittleren Position innerhalb des Hohlkörpers 6 befindet. Die Hohlnadel 12 wurde dabei vom Kolben 18 mitgenommen und befindet sich somit nunmehr vollständig innerhalb des Hohlkörpers 6.

Um die oben erläuterte Funktionsweise auf besonders zuverlässige und in mehrfacher Hinsicht vorteilhafte Weise zu erreichen, sind die Komponenten in diversen Details spezifisch ausgestaltet, wobei die nachfolgend beschriebenen Ausgetaltungen jeweils sowohl als eigenständig erfinderisch als auch in beliebiger Kombination miteinander als erfinderisch angesehen werden.

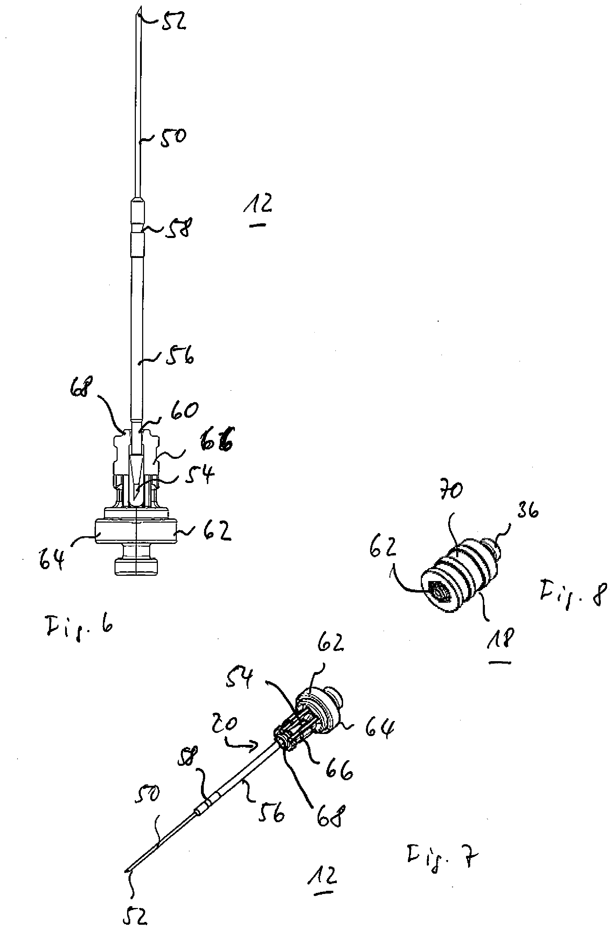

So ist beispielsweise die Hohlnadel 12 gemäß nachfolgender Beschreibung eigenständig erfinderisch ausgeführt. Wie der vergrößerten Darstellung in den Fig. 6 (seitliche Ansicht) und Fig. 7 (perspektivische Ansicht) entnehmbar ist, umfasst die Hohlnadel 12 in eigenständig erfinderischer Ausgestaltung ein Nadelrohr 50 aus Metall, das an seinen beiden Enden jeweils eine Nadelspitze 52, 54 bildet. Die Materialwahl für das Nadelrohr 50 ist dabei bevorzugt im Hinblick auf gängige Erfordernisse für medizinische Anwendungen geeignet erfolgt, wobei besonders bevorzugt ein auch für Standard-Nadeln verwendbares rostfreies Material vorgesehen ist. In seinem mittteleren Längenbereich ist das Nadelrohr 50 ummantelt ausgeführt und von einem Kunststoffmantel 56 umgeben. Als Material für den Kunststoffmantel 56 ist dabei vorzugsweise ein Polyamid (PA12), ganz besonders bevorzugt das im Handel unter der Bezeichnung Vestamid Care ML 17 erhältliche, vorgesehen. Der Kunststoffmantel 56 wird dabei in einer ganz besonders bevorzugten, ebenfalls als eigenständig erfinderisch angesehenen Ausgestaltung auf das Nadelrohr 50 aufgespritzt, nachdem dieses in der Art einer Oberflächenaktivierung einer Plasmavorbehandlung unterzogen wurde. Damit ist eine besonders gute Haftung des den Kunststoffmantel 56 bildenden Kunststoffs auf der Nadel 50 erreichbar. Im Kunststoffmantel 56 sind zwei Haltenuten 58, 60 ausgeformt, die die oben beschriebene Funktionsweise ermöglichen sollen.

Die erste Haltenut 58 ist dabei für die vorübergehende Fixierung der Nadel 12 in der Lagermuffe 14 des Nadelhalters 10 vorgesehen. Dazu ist innerhalb der Lagermuffe 14 eine zugeordnete umlaufende Rastlippe vorgesehen, die bei montierter und in die Lagermuffe 14 ordnungsgemäß eingeschobener Nadel 12 in die Haltenut 58 eingreift und diese in Längsrichtung fixiert. Gemäß einer grundsätzlich als eigenständig erfinderisch angesehenen Ausgestaltung sind die Dimensionierungen der Haltenut 58 und der Rastrille dabei vorteilhafterweise derart gewählt, dass unter Berücksichtigung der Verformbarkeit des Materials des Kunststoffmantels 56 und/oder einer eventuellen Klebekraft durch die Materialpaarung des Materials des Kunststoffmantels 56 und des diesen umgebenden Materials der Lagermuffe 14 die Halte- oder Losbrechkraft der solchermaßen eingerasteten Nadel 12 in Längsrichtung einerseits ausreichend groß ist, so dass die Nadel 12 entsprechend der oben beschriebenen Vorgehensweise in die Haut des Patienten eingestochen werden kann, andererseits aber auch ausreichend klein ist, so dass die beschriebene Retraktionsbewegung der Nadel 12 zum Inneren des Hohlkörpers 6 hin durchgeführt werden kann. Gegebenenfalls kann das Profil der Haltenut 58 auch entsprechend asymmetrisch ausgeführt sein, mit einem vergleichsweise steilen Flankenwinkel an ihrer der dem Innenraum zugewandten Spitze 54 zugewandten Seite und einem vergleichsweise flachen Flankenwinkel an ihrer der freiliegenden Spitze 52 zugewandten Seite.

Die zweite Haltenut 60 ist hingegen für eine entsprechende Verrastung im Kolben 18 vorgesehen. In den Figs. 6, 7 ist die Nadel 12 im in den Kolben 18 eingesteckten Zustand gezeigt. Der Kolben 18 ist dabei seinerseits mehrteilig ausgeführt und umfasst den in den Figs. 6, 7 ebenfalls gezeigten erfindungsgemäßen Nadelhalter 62. Der Nadelhalter 62 ist dabei als Kunststoffteil ausgeführt und besteht im Ausführungsbeispiel im Hinblick auf die erforderlichen Haltekräfte und die bei bestimmungsgemäßem Gebrauch erwarteten mechanischen Belastungen, aber auch im Hinblick auf zulassungsbedingte Erfordernisse aus dem unter der Bezeichnung "Bormed^{™}" (HD810MO, ISO 10993 Information (Biocompatibility)) erhältlichen Polypropylen.

Wie der Darstellung in Fig. 6, besonders gut aber auch der perspektivischen Ansicht in Fig. 7 entnehmbar ist, umfasst der Nadelhalter 62 einen an einen Grundkörper 64 angeformten Haltebügel 66, der das eigentliche, das Aufnahmeloch 20 bildende Nadellager 68 trägt. Das Nadellager 68 ist dabei, analog der oben beschriebenen Lagermuffe 14, innenseitig mit einer zugeordneten umlaufenden Rastlippe versehen, die bei in das Nadellager 68 eingeschobener Nadel 12 in die zweite Haltenut 60 eingreift und diese in Längsrichtung fixiert. Gemäß einer grundsätzlich ebenfalls als eigenständig erfinderisch angesehenen Ausgestaltung sind die Dimensionierungen der Haltenut 60 und der dieser zugeordneten Rastrille im Nadellager 68 dabei vorteilhafterweise derart gewählt, dass unter Berücksichtigung der Verformbarkeit des Materials des Kunststoffmantels 56 und/oder einer eventuellen Klebekraft durch die Materialpaarung des Materials des Kunststoffmantels 56 und des diesen umgebenden Materials des Nadellagers 68 die Halteoder Losbrechkraft der solchermaßen eingerasteten Nadel 12 in Längsrichtung größer ist als die enstprechende Halte- oder Losbrechkraft der Haltenut 58 in der Lagermuffe 14, so dass bei der Retraktionsbewegung des Kolbens 18 die Nadel 12 von diesem zum Inneren des Hohlkörpers 6 mitgenommen wird.

Durch die Ausgestaltung des Haltebügels 66 wird zudem innerhalb des Kolbens 18 ein Freiraum geschaffen, der in der Endphase der Ausbringung des Wirkstoffs, bei der die Nadelspitze 54 schon in das Aufnahmeloch 20 eingedrungen und somit für den Wirkstoff nicht mehr ohne weiteres zugänglich ist, in der Art eines Bypasses das Einströmen des Wirkstoffs über die Nadelspitze 54 in das Nadelrohr 50 ermöglicht. Der Zustrom kann dabei beidseitig des Haltebügels 66 in den Freiraum hinein erfolgen.

Wie bereits ausgeführt, ist in einer weiteren, ebenfalls als eigenständig erfinderisch angesehenen Ausgestaltung der Kolben 18 mehrteilig ausgeführt. Wie der vergrößerten Ansicht gem. Fig. 8 entnehmbar ist, ist dabei zusätzlich zu dem bereits beschriebenen Nadelhalter 62 für die Nadel 12 ein diesen umgebender Kolbenmantel 70 vorgesehen. Der Kolbenmantel 70 besteht dabei unter Berücksichtigung zulassungsbedingter Erfordernisse aus herkömmlichem Gummi, besonders bevorzugt aus dem von der Firma Kreiburg unter der Bezeichnung TM4RST (MC/RS Series) erhältlichen Material. Der Kolbenmantel 70 ist dabei derart geformt, dass er bei eingebrachtem Nadelhalter 62 beidseitig des Haltebügels 66 Zuströmkanäle für den Wirkstoff freilässt, so dass im Sinne einer Vermeidung oder Minimierung des Totvolumens der Bypass für den angestrebten Nullvolumen-Ausstoß gebildet wird.

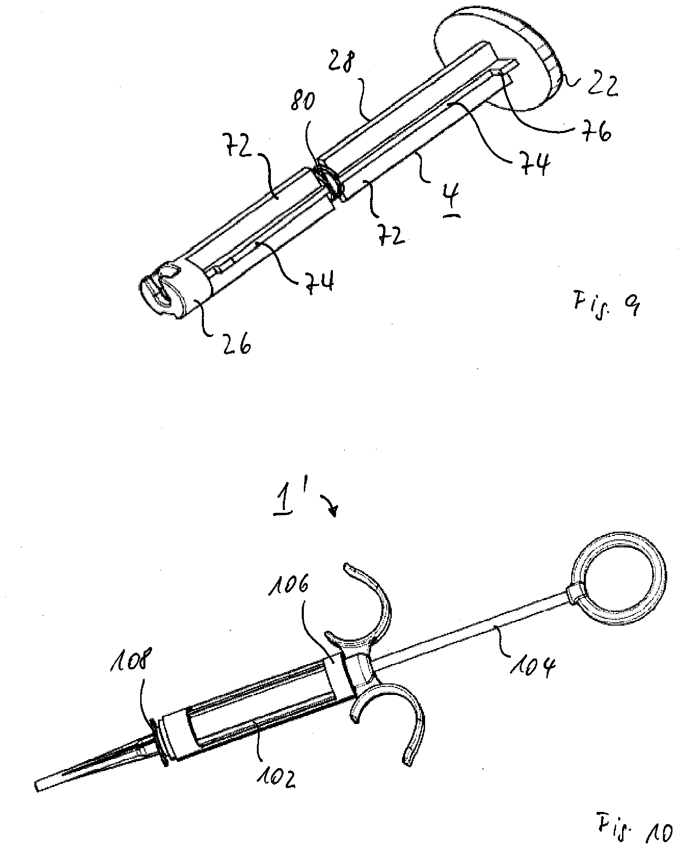

Um die zur Duchführung der vorstehend beschriebenen Abfolge bei der Bedienung der Spritze 1 zur Vorgabe der jeweils vorgesehenen Endpositionen von Kolben 18 bzw. Schaft 28 innerhalb des Hohlkörpers 6 benötigten Anschläge bereitzustellen, ist eine spezifische, ebenfalls als eigenständig erfinderisch angesehene Ausgestaltung des Schafts 28 des Betätigungsstößels 4 vorgesehen. Zur Verdeutlichung ist dieser vergrößert in Fig. 9 dargestellt. Wie dieser Darstellung entnehmbar ist, wird der Schaft 28 im Wesentlichen durch zwei gekreuzte, im Querschnitt ein Kreuz bildende Schaftrippen 72, 74 gebildet. Die Oberkanten der Schaftrippen 72, 74 verlaufen in Längsrichtung des Betätigungsstößels 4 gesehen im Wesentlichen geradlinig und über weite Teile der Gesamtlänge des Betätigungsstößels 4 im Wesentlichen parallel zu dessen Längsrichtung. An einer Position relativ nahe zur Drückerplatte 22 weist die Schaftrippe 74 jedoch einen Vorsprung auf, so dass sich hier eine Kante 76 bildet. Die Kante 76 schlägt dabei bei der zur Befüllung der Spritze 1 zunächst vorgesehenen Einwärtsbewegung des Betätigungsstößels 4 in den Hohlkörper 6 hinein an der Deckelplatte 30 an und begrenzt somit diese Einwärtsbewegung.

Nach der anschließend vorgesehenen Rotationsbewegung des Betätigungsstö-ßels 4 um seine Längsachse um den bevorzugt vorgesehenen Verdrehwinkel von etwa 90° wird die die Kante 76 aufweisende erste Schaftrippe rotatorisch mit einer von der Außenkontur 42 des Aufnahmelochs 40 gebildeten Führungsnut 78 in Überlappung gebracht, so dass in dieser Orientierung der Betätigungsstößel 4 noch weiter zum distalen Ende 8 des Hohlkörpers 6 hin verschoben werden kann.

Des Weiteren ist in den Schaftrippen 72, 74 noch eine Kerbe 80 vorgesehen, in die bei entsprechender Positionierung eine in der Deckelplatte 30 angeordnete Sicherungsfeder 82 einrasten kann. Dies dient dazu, die eingenommene Position nach erfolgter Retraktion der Nadel 12 in das Innere des Hohlkörpers 6 hinein zu fixieren, so dass die Nadel 12 dort gesichert verbleibt.

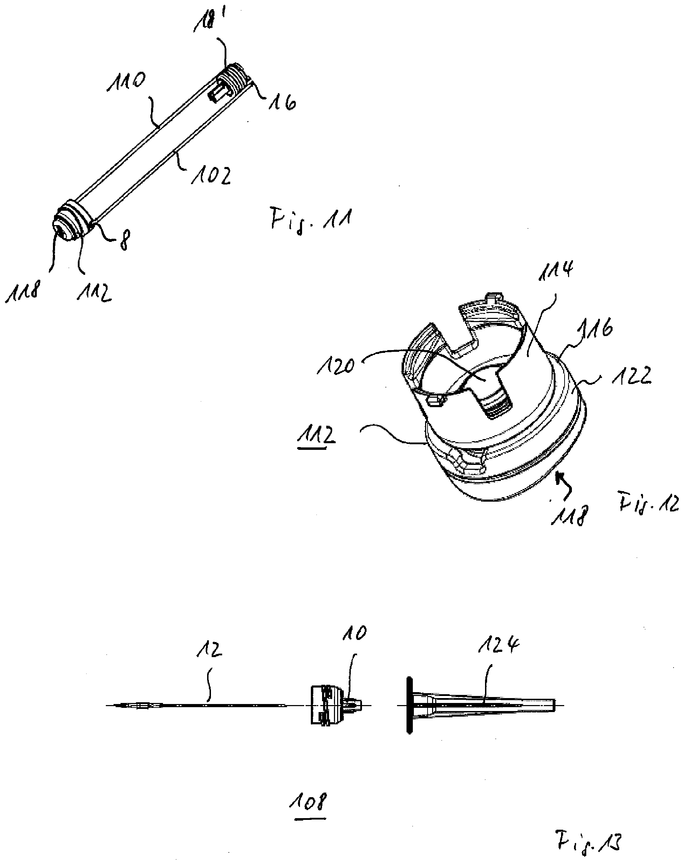

Eine alternative, als eigenständig erfinderisch angesehene medizinische Spritze 1 ' mit Nadelretraktion ist in den Figs. 10 ff. gezeigt. Diese medizinische Spritze 1' ist insbesondere für mit Wirkstoff vorbefüllte Kartuschen vorgesehen und umfasst im Wesentlichen drei Baugruppen, nämlich eine Kartuschen- oder Patroneneinheit 102, einen mit einem Betätigungsstößel 104 versehenen Rahmen 106 und einen Nadelkopf 108. Die in Fig. 11 vergrößert gezeigte Kartuschen- oder Patroneneinheit 102 ist in diesem Ausführungsbeispiel mit dem Wirkstoff vorbefüllt und umfasst den eigentlichen, als zylindrischen Hohlkörper ausgestalteten Kartuschenkörper 110, der an seinem proximalen Ende 16 mit dem Kolben 18' verschlossen ist. Am distalen Ende 8 ist er hingegen von einer aufgeclipten oder aufgesteckten Kappe 112 verschlossen, wie sie in Fig. 12 gezeigt ist.

Die als eigenständig erfinderisch angesehene Kappe 112 gem. Fig. 12 umfasst einen mit einer umlaufenden, als Clip für die Verbindung mit dem Rand des Kartuschenkörpers 110 vorgesehenen Seitenschürze 114 versehenen Deckel 116. Dieser umfasst ein zentrales Aufnahmeloch 118 für die Hohlnadel 12, und er ist innenseitig mit einer vorzugsweise aufgespritzten Dichtung 120 aus geeignet gewähltem Material, insbesondere TPE, versehen. Die Seitenschürze 114 ist von einem verschiebbaren Überwurfmantel 122 umgeben. Nach dem Aufclippen oder Aufsprengen auf den Rand des Kartuschenkörpers 110 kann der Überwurfmantel 122 über die mit Rasthaken versehene Seitenschürze 114 geschoben werden, so dass die Verhakung fixiert und die Verbindung damit gesichert wird.

Der in Fig. 13 in Explosionsdarstellung gezeigte Nadelkopf 108 umfasst den Nadelhalter 10, die Hohlnadel 12 und eine Nadelschutzkappe 124. Zusätzlich ist eine Versiegelung 126 zur Wahrung der Sterilität vorgesehen.

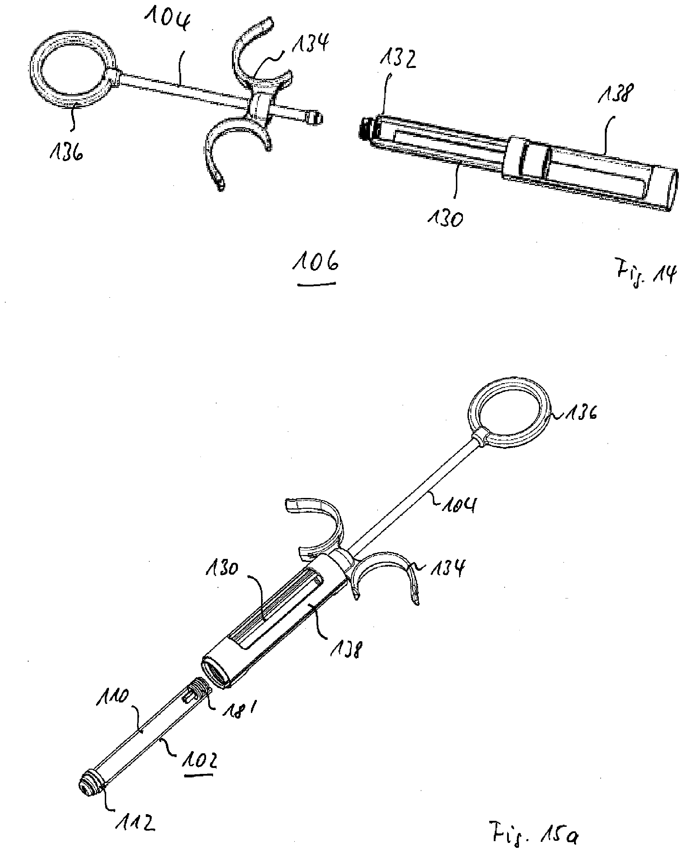

Der Spritzenrahmen 106 umfasst, wie dies aus Fig. 14 ersichtlich ist, einen zur Aufnahme der Kartuscheneinheit 102 vorgesehenen, endseitig offenen, vorzugsweise zylindrisch oder halbzylindrisch ausgestalteten Kammerkörper 130, an dessen proximalem Ende 132 zusätzlich zu einer Fingerauflage 134 der in diesem Ausführungsbeispiel endseitig mit einer Fingerlasche 136 versehene Betätigungsstößel 104 angeordnet ist. Der Kammerkörper 130 ist dabei von einer in Längsrichtung verschiebbaren Nadelschutzhülse 138 umgeben.

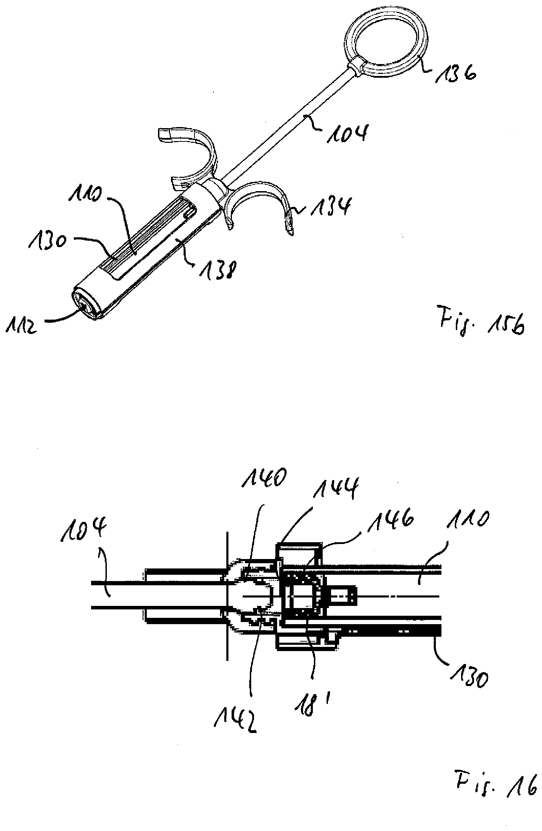

Bei der Benutzung der medizinischen Spritze 1' wird zunächst, wie dies in Fig. 15 gezeigt ist, der befüllte und endseitig veschlossene Kartuschenkörper 110 durch dessen offenes Ende in den Kammerkörper 130 vollständig eingebracht. Fig. 15a zeigt dabei die Bauteile unmittelbar vor, Fig. 15b die Bauteile unmittelbar nach dieser Einbringung. In diesem Ausführungsbeispiel ist der Kolben 18' dabei in als eigenständig erfinderisch angesehener Weise für eine besonders einfache Verkupplung mit dem Betätigungsstößel 104 ausgelegt. Wie der Darstellung im Längsschnitt gemäß Fig. 16 entnehmbar ist, weist der Betätigungsstößel 104 dabei endseitig eine Verdickung 140 mit umlaufender Rastrille 142 auf. Korrespondierend dazu ist der Kolben 18' in diesem Ausführungsbeispiel mit einem Aufnahmekanal 144 versehen, in den die Verdickung 140 einschiebbar ist. Korrespondierend zur Rastrille 142 ist der Aufnahmekanal 144 mit einer umlaufenden Rastlippe 146 versehen, die bei vollständiger Einbringung mit der Rastrille 142 verrastet. Damit ist der Kolben 18' mit dem Betätigungsstößel 4' verbunden.

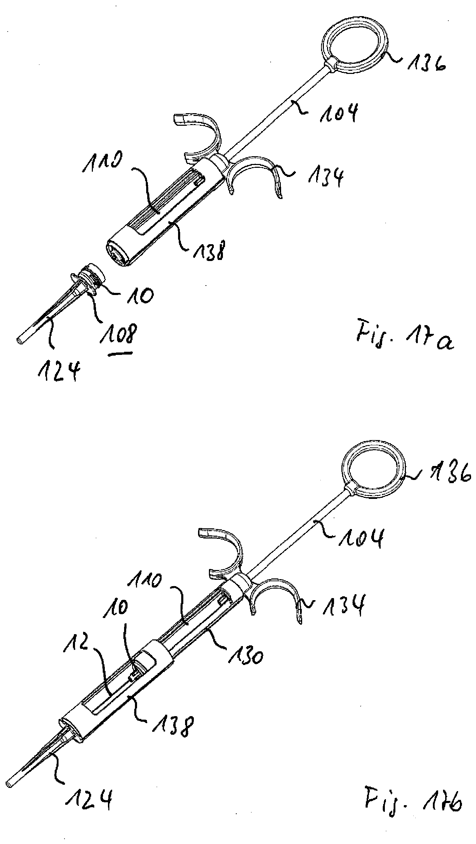

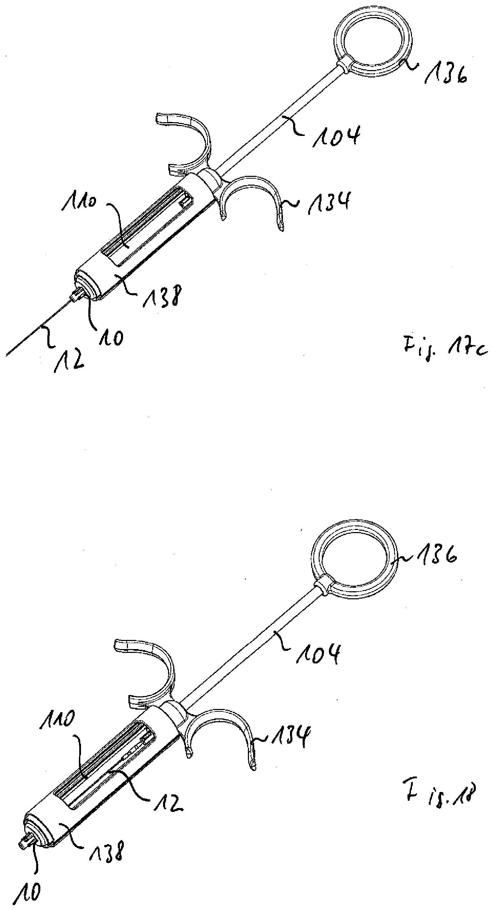

In Fig. 17 ist gezeigt, wie die medizinische Spritze 1'anschließend einsatzfertig gemacht wird. Dazu wird zunbächst nach der Einbringung des Kartuschenkörpers 110 in den Kammerkörper 130, wie dies in Fig. 17a, gezeigt ist, der Nadelkopf 108 angebracht. Dies kann auf besonders einfache Weise durch Anschrauben erfolgen; dazu ist vorzugsweise der Nadelhalter 10 in diesem Ausführungsbeispiel außenseitig mit einem vorzugsweise zweigängigen Luergewinde versehen, das mit einem korrespondierenden Innengewinde im Endbereich des Kammerkörpers 130 zusammenwirkt. Bei dieser Montage durchsticht die innenseitige Nadelspitze 54 im Aufnahmeloch 118 die Dichtung 120 und ragt somit in den Innenraum des Kartuschenkörpers 110 hinein. Anschließend wird, wie dies in Fig. 17b gezeigt ist, die Nadelschutzhülse 138 zur Nadel 12 hin verschoben, so dass sie die Nadelschutzkappe 124 abhebt. Diese wird somit entfernt, ohne dass der Benutzer Gefahr läuft, sich zu stechen, und anschließend wird die Nadelschutzhülse 138 wieder zurückgezogen, so dass die Nadel 12 nunmehr einsatzbereit ist und frei liegt, gezeigt in Fig. 17c.

Anschließend kann durch Drücken des Betätigungsstößels 104 der Wirkstoff verabreicht werden, wobei analog zur oben bereits beschriebenen Funktionsweise nach vollständiger Abgabe des Wirkstoffs das "connecting" der Nadel 12 mit dem Kolben 18' und die Einbringung des entsprechenden Teilbereichs der Hohlnadel 12 in das Aufnahmeloch 20 des Kolbens 18' erfolgt. Anschließend wird auch hier vom Bediener der Betätigungsstößel 104 wieder zurückgezogen. Damit wird auch hier der mit dem Betätigunsstößel 104 verbundene Kolben 18' und mit diesem die eingefasste Nadel 12 mitgenommen, so dass diese vollständig in den Kartuschenkörper 110 hineingezogen wird. Wie der Darstellung dieses Zustands in Fig. 18 entnehmbar ist, kann dann der Nadelhalter 10 wieder abgeschraubt werden. Anschließend kann der Benutzer durch Ziehen am Betätigungsstößel 104 dessen Verrastung mit dem Kolben 18' wieder lösen, so dass der nunmehr die Nadel 12 vollständig umschließende Kartuschenkörper 110 aus dem Kammerkörper 130 entnommen werden kann.

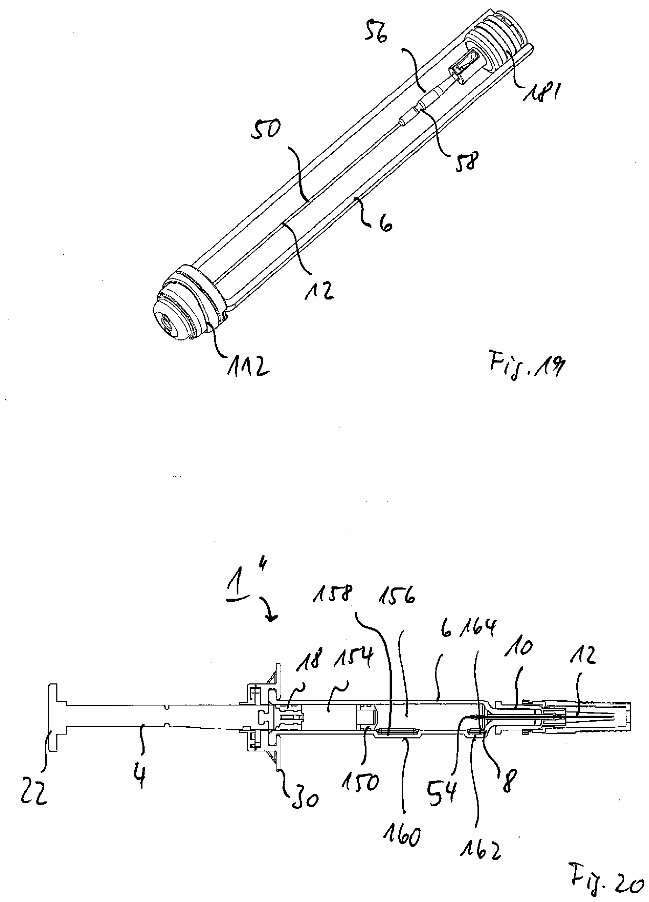

Der somit zur sicheren Entsorgung bereitstehende, die Nadel 12 vollständig umschließende Kartuschenkörper 110 ist in Fig. 19 gezeigt. Nach dessen Entnahme aus dem Kammerkörper 130 kann der als Mehrwegsystem ausgelegte und für eine mehrfache Verwendung vorgesehene Rahmen 106 einer weiteren Nutzung zugeführt werden.

Eine alternative, ebenfalls als eigenständig erfinderisch angesehene Ausführungsform einer medizinischen Spritze 1" mit in der Art eines Retraktionssystems ausgeführtem passiven Nadelschutz der genannten Art ist in Fig. 20 im Längsschnitt gezeigt. Fig. 20 zeigt die medizinische Spritze 1" dabei vor Ausbringung des Wirkstoffs. In dieser alternativen Ausführungsform ist die medizinische Spritze 1" mit einem Doppelkammer- oder Doppelkolbensystem ausgestattet. In einer ansonsten zur vorgenannten Ausführungsform weitgehend gleichen Bauweise weist die medizinische Spritze 1" dabei zusätzlich zum Kolben 18 innerhalb des Hohlkörpers 6 noch einen weiteren, vorgelagerten Kolben 150 auf. Durch den Zwischenraum zwischen dem Kolben 18 und dem weiteren Kolben 150 wird dabei eine erste Wirkstoffkammer 154 gebildet, und zwischen dem weiteren Kolben 150 und dem apikalen Ende 8 des Hohlkörpers 6 befindet sich dann die zweite Wirkstoffkammer 156.

Ein solches Doppelkammersystem wird insbesondere eingesetzt für flüssige und/oder lyophilisierte (gefriergetrocknete) oder pulverförmig vorliegende Medikamente, die vor der Verabreichung gelöst werden müssen. Solche Doppelkammer-Systeme sind somit eine besonders geeignete Lösung für lyophilisiert /flüssig oder flüssig/flüssig Wirkstoffkombinationen. Das System bietet eine Vielzahl von Vorteilen für empfindliche Injektionsmedikamente.

In der ersten Wirkstoffkammer 154 wird dabei beispielsweise das Lösungsmittel vorgehalten, wohingegen sich der eigentliche, z. B. gefriergetrocknete oder pulverförmige Wirkstoff in der zweiten Wirkstoffkammer 156 befindet. Bei der Verabreichung des Wirkstoffs wird durch Betätigung des Betätigungsstößels 4 dabei zunächst das in der ersten Wirkstoffkammer 154 bereitgestellte Lösungsmittel über einen in der Gehäusewand des Hohlkörpers 6 angeordneten, von einer Ausformung 158 im Gehäusemantel gebildeten Bypasskanal 160 am weiteren Kolben 150 vorbei in die zweite Wirkstoffkammer 156 eingebracht. Dort löst es den dort vorgehaltenen Wirkstoff, so dass dieser verabreichungsfertig ist. Anschließend wird durch weitere Betätigung des Betätigungsstößels 4 der Kolben 18 bis zum Anschlag an den Kolben 150 bewegt, und anschließend werden die Kolben 18, 150 gemeinsam weiterbewegt, so dass der in der zweiten Wirkstoffkammer 156 befindliche, nummehr gelöste Wirkstoff über die Nadel 12 ausgebracht wird.

Sobald der weitere Kolben 150 die Nadel 12 erreicht und diese in den Kolbenkörper eindringt, wird der restliche Wirkstoff ausgebracht und anschließend das Retraktionssystem gemäß der vorstehend beschriebenen Funktionsweise ausgelöst. Die Verringerung des Totvolumens im Endbereich des Hohlkörpers 6 wird dabei durch eine weitere, einen Bypasskanal 162 bildende Ausformung 164 ermöglicht, über die die Restmenge an Wirkstoff vergleichbar zur oben bereits beschriebenen Ausführung dem Ende 54 der Nadel 12 zuströmen kann.

Auch in dieser alternativen Ausführunsgform der medizinischen Spritze 1" könnte der Nadelhalter 10 einstückig mit dem das Spritzengehäuse bildenden Hohlkörper 6 ausgeführt sein. Im vorliegend dargestellten, als eigenständig erfinderisch angesehenen Ausführungsbeispiel ist der Nadelhalter 10 aber als separates Bauteil ausgestaltet und auf den das Spritzengehäuse bildenden Hohlkörper 6 bzw. den Spritzenkonus aufgesteckt oder aufsteckbar ausgeführt. Alternativ könnte der Nadelhalter 10 auch mittels eines Gewindes, vorliegend insbesondere eines Luer-Gewindes, an den Hohlkörper 6 anschraubbar ausgeführt sein.

### Bezugszeichenliste

- 1, 1', 1": Medizinische Spritze
- 2: Kartuschen- oder Patroneneinheit
- 4: Betätigungsstößel
- 6: Hohlkörper
- 8: distales Ende
- 10: Nadelhalter
- 12: Hohlnadel
- 14: Lagermuffe
- 16: proximales Ende
- 18: Kolben
- 20: Aufnahmeloch
- 22: Drückerplatte
- 24: Ende
- 26: Kuppelelement
- 28: Schaft
- 30: Deckelplatte
- 36: Befestigungsstift
- 38: Langloch
- 40: Aufnahmeloch
- 42: Außenrand
- 50: Nadelrohr
- 52,54: Nadelspitze
- 56: Kunststoffmantel
- 58,60: Haltenut
- 62: Nadelhalter
- 64: Grundkörper
- 66: Haltebügel
- 68: Nadellager
- 70: Kolbenmantel
- 72, 74: Schaftrippe
- 76: Kante
- 78: Führungsnut
- 80: Kerbe
- 82: Sicherungsfeder
- 102: Kartuschen- oder Patroneneinheit
- 104: Betätigungsstößel
- 106: Rahmen
- 108: Nadelkopf
- 110: Kartuschenkörper
- 112: Kappe
- 114: Seitenschürze
- 116: Deckel
- 118: Aufnahmeloch
- 120: Dichtung
- 122: Überwurfmantel
- 124: Nadelschutzkappe
- 126: Versiegelung
- 130: Kammerkörper
- 132: proximales Ende
- 134: Fingerauflage
- 136: Fingerlasche
- 138: Nadelschutzhülse
- 140: Verdickung
- 142: Rastrille
- 144: Aufnahmekanal
- 146: Rastlippe
- 150: Kolben
- 154, 156: Wirkstoffkammer
- 158, 164: Ausformung
- 160, 162: Bypasskanal
- D: maximaler Abstand
- d: minimaler Abstand
- dE: Endabstand

## Patentansprüche

1. Spritze (1) mit Nadelschutz, mit einem über einen Betätigungsstößel (4) im Innenraum eines Spritzenkörpers (6) verschiebbaren Kolben (18), an dem ein zur Aufnahme der Spritzennadel (12) vorgesehener Nadelhalter (62) angeordnet ist, wobei der Nadelhalter (62) einen an einen Grundkörper (64) angeformten Haltebügel (66) aufweist, der das eigentliche, das Aufnahmeloch (20) für die Spritzennadel (12) bildende, innenseitig mit einer umlaufenden Rastlippe versehene Nadellager (68) trägt, die bei in das Nadellager (68) eingeschobener Spritzennadel (12) in eine außenseitig an der Spritzennadel (12) angeordnete Haltenut (60) eingreift und die Spitzennadel (12) somit in Längsrichtung fixiert, und wobei der Haltebügel (66) unter Bildung eines Freiraums innerhalb des Kolbens (18) derart ausgestaltet ist, dass der Freiraum in der Endphase der Ausbringung des Wirkstoffs, bei der die Nadelspitze (54) schon in das Aufnahmeloch (20) eingedrungen und somit für den Wirkstoff nicht mehr ohne weiteres zugänglich ist, in der Art eines Bypasses das Einströmen des Wirkstoffs über die Nadelspitze (54) in das Nadelrohr (50) der Spritzennadel (12) ermöglicht.

2. Spritze (1) nach Anspruch 1, deren Nadelhalter (62) als Kunststoffteil, vorzugsweise aus Polypropylen, gefertigt ist.

3. Spritze (1) nach Anspruch 1 oder 2, deren Nadelhalter (62) von einem Kolbenmantel (70) umgeben ist, wobei der Kolbenmantel (70) derart geformt ist, dass er bei eingebrachtem Nadelhalter (62) seitlich des Haltebügels (66) eine Anzahl von Zuströmkanälen für den Wirkstoff freilässt.

4. Spritze (1) nach Anspruch 3, deren Kolbenmantel (70) aus Gummi besteht.

## Claims

1. A syringe (1) having a needle guard, with a piston (18) which can be displaced in the inner chamber of a syringe body (6) by means of an actuating plunger (4), on which piston a needle retainer (62) is disposed which is provided to receive the syringe needle (12), wherein the needle retainer (62) has a retaining bracket (66) formed on a main body (64) and which carries the actual needle seat (68) which forms the receiving aperture (20) for the syringe needle (12) and which is provided with a circumferential locking lip on the inside which, when the syringe needle (12) has been inserted into the needle seat (68), engages in a retaining groove (60) disposed on the outside of the syringe needle (12) and thereby fixes the syringe needle (12) in the longitudinal direction, and wherein, because of the formation of a free space inside the piston (18), the retaining bracket (66) is configured in a manner such that in the final phase of dispensing the active ingredient during which the needle tip (54) has already penetrated into the receiving aperture (20) and therefore the active ingredient no longer has direct access to it, the free space enables the active ingredient to flow via the needle tip (54) into the needle tube (50) of the syringe needle (12) in the manner of a bypass.

2. The syringe (1) as claimed in claim 1, the needle retainer (62) being produced as a plastic part, preferably from polypropylene.

3. The syringe (1) as claimed in claim 1 or claim 2, the needle retainer (62) being surrounded by a piston sheath (70), wherein the piston sheath (70) is formed in a manner such that when the needle retainer (62) has been inserted, a number of inflow channels for the active ingredient are exposed on the side of the retaining bracket (66).

4. The syringe (1) as claimed in claim 3, the piston sheath (70) consisting of rubber.

## Revendications

1. Seringue (1) à protection d'aiguille, comprenant un piston (18) pouvant être poussé dans l'espace intérieur d'un corps de seringue (6) par un poussoir d'actionnement (4), sur lequel est disposé un porte-aiguille (62) prévu pour recevoir l'aiguille de seringue (12), dans lequel le porte-aiguille (62) présente un étrier de retenue (66) formé sur un corps de base (64), qui porte le palier d'aiguille (68) à proprement dit, formant le trou de réception (20) pour l'aiguille de seringue (12) et doté à l'intérieur d'une lèvre d'encliquetage périphérique, qui se met en prise dans une rainure de retenue (60) disposée sur l'extérieur de l'aiguille de seringue (12) lorsque l'aiguille de seringue (12) est rentrée dans le palier d'aiguille (68) et fixe ainsi l'aiguille de seringue (12) dans le sens longitudinal, et dans lequel l'étrier de fixation (66), par formation d'un espace libre à l'intérieur du piston (18), est ainsi formé que l'espace libre dans la phase terminale de l'apport de substance active dans laquelle la pointe d'aiguille (54) a déjà pénétré dans le trou de réception (20) et n'est donc plus aisément accessible pour la substance active, permet l'entrée de la substance active dans le tube d'aiguille (50) de l'aiguille de seringue (12) par le biais de la pointe d'aiguille (54), à la manière d'une dérivation.

2. Seringue (1) selon la revendication 1, dont le porte-aiguille (62) est fabriqué en tant que pièce en plastique, de préférence en polypropylène.

3. Seringue (1) selon la revendication 1 ou 2, dont le porte-aiguille (62) est entouré par une jupe de piston (70), dans lequel la jupe de piston (70) est ainsi formée que lorsque le porte-aiguille (62) est inséré, il libère un nombre de canaux d'amenée pour la substance active sur le côté de l'étrier de support (66).

4. Seringue (1) selon la revendication 3, dont la jupe de piston (70) est en caoutchouc.
